# EUROPEAN PATENT SPECIFICATION

(11) **EP 1 345 531 B1**
(45) Date of publication and mention of the grant of the patent: **21.02.2007**
(21) Application number: 01270274.2
(22) Date of filing: 11.12.2001
(51) Int. Cl.: A61B 5/15

(54) **LANCET FOR SKIN PRICKERS**
LANZETTE FÜR HAUTSTICHE
LANCETTE DESTINEE A PIQUER LA PEAU

(30) Priority: 14.12.2000 GB 0030489
(43) Date of publication of application: 24.09.2003
(73) Proprietor: OWEN MUMFORD LIMITED, Woodstock, Oxfordshire OX20 1TU (GB)
(72) Inventor: BICKNELL, Stephen, Shipston on Stour Warwickshire CV36 4PJ (GB)
(74) Representative: Newell, William Joseph
(86) International application number: PCT/GB2001/005433
(87) International publication number: WO 2002/047550

(56) References cited:
- EP-A- 0 589 186
- WO-A-96/02189

## Description

This invention relates to lancets for skin prickers. These draw a small drop of blood for analysis, and they are widely used by diabetics, for example, who need to know their sugar level.

One of the intricate and complex tasks faced in manufacturing such devices is the precision machining and grinding required to form a point on the needle tip. This typically requires three flats to be ground onto the needle tip. This is exacerbated by the desire to make the cross-section of the needle tip as small as possible to achieve a sharper point and therefore give a less noticeable prick. For an existing design such as that shown in EP-B-0 858 289 the needle blanks are taped up, i.e. attached in a row between two layers of sticky tape, and then ground in several passes to produce a flat at one end of each blank. This portion of reduced cross-sectional area then has a point formed at its tip. However, while this can give a really sharp point, the needle is of small diameter and there are difficulties arising from such lack of size during the moulding processes. It would be easier to precision machine and mould around a more substantial body, especially in high volume manufacture.

According to one aspect of the present invention there is provided a lancet for a skin pricker, the lancet having a needle encased, apart from its tip, in a body of plastics material, characterised in that the needle has a larger diameter portion of generally circular cross-section within the lancet body but with a coaxial reduction in diameter before emerging at the tip, to provide a smaller diameter portion of generally circular cross-section, and in that the larger diameter portion of the needle has a diameter in the range 0.6 to 1.0 mm.

With this construction the diameter of the main body of the needle can be sufficiently large to enhance the rigidity of the lancet main body- It can also facilitate handling of the needle blank during its manufacturing and moulding stages.

The reduction in diameter may either be by an abrupt step or by a tapered shoulder.

Often a breakaway cap will be integrally moulded with the body to encase the needle tip. This is for safety and hygiene, and the cap may also serve as the part which the user manipulates to cock the lancet firing device. When the cap is removed the step or shoulder will prevent any tendency of the needle to shift forwards and expose more tip than intended.

The larger diameter portion of the needle preferably has a diameter of about 0.8 mm (21 gauge). The reduced diameter portion of the needle preferably has a diameter in the range 0.2 to 0.5 mm, and the optimum is likely to be in the range 0.3 to 0.4 mm.

According to another aspect of the present invention there is provided a method of producing a lancet for a skin pricker, the method comprising:
providing a generally cylindrical needle blank, reducing the diameter of one end portion of the blank, forming a point on the free end or tip of said one end portion, thus creating a needle,
introducing the needle into a mould, and
introducing plastics material into said mould to form a lancet body around the needle apart from its tip,
characterised in that the reduced diameter portion (10) is of generally circular cross-section and coaxial with the larger diameter portion, and in that the reduced diameter portion has a diameter in the range from 0.6 mm to 1.0 mm.

The tip may have a breakaway plastics cap moulded around it in the same operation as the moulding of the body, as mentioned above.

The reduction of said one end portion of the blank may be by centreless cylindrical grinding, rolling or turning.

Needle blanks of a diameter corresponding to that of the main body of the finished needle may be supplied to an automated machining or working device, which machines or works the blank to produce the reduced diameter portion to form the needle tip. This is achieved without the laborious process of taping up needle blanks as previously. Since the part-formed blank (prior to needle tip formation) resulting from this process is still rotationally symmetric, further handling and manufacture of the needle and the complete lancet is facilitated.

For a better understanding of the invention, one embodiment will now be described, by way of example only, with reference to the accompanying drawing, in which:-
Figure 1 is a side view of a lancet in a pre-used condition;
Figure 2 is another side view of the lancet taken from a direction perpendicular to that of Figure 1;
Figure 3 is a view of the lancet similar to that of Figure 2 but showing the breakaway cap removed,
Figure 4 is a cross-section taken on lines iv - iv of Figure 2, and
Figure 5 is a side view of a needle for this lancet.

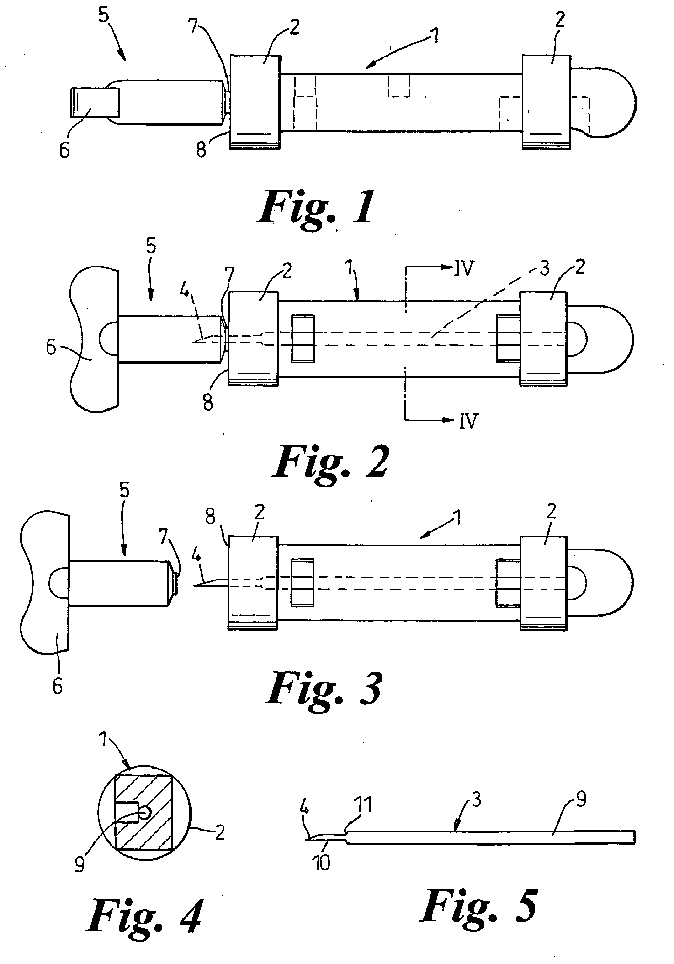

The lancet has an elongate body 1 of moulded plastics material. As is apparent in Figure 4, the body is of generally rectangular section with two circular collars 2 at opposite ends. Coaxially encased within the body is a needle 3, shown in dotted lines in Figures 3 and 4 and fully in Figure 5, and which projects in a sharp tip 4 at the forward end.

The tip 4 is initially encased in a cap 5 which is in the form of a clock key with a transverse head 6. The cap 5 is integrally moulded with the body 1 and connects to it by a neck 7 which is weak enough to be sheared off from the end surface 8 of the body by a twisting action. Such a cap may not always be provided.

The needle has a cylindrical main body portion 9 over most of its length, the remainder being a co-axial needle tip portion 10 at one end of reduced diameter produced by a process such as centreless cylindrical grinding, rolling or turning. The free extremity of this portion 10 is ground to provide the sharp tip 4. In this embodiment the outer diameter of the portion 9 is of the order of 0.8 millimetres (21 gauge) with the diameter of the needle tip portion 10 being of the order of 0.3 to 0.4 millimetres, but the invention is not limited to these dimensions or to similar proportions. It is considered reasonable for the larger diameter to be in the range 0.6 to 1.0 millimetres and the reduced one to be in the range 0.2 to 0.5 millimetres. Between the portions 9 and 10 is a shoulder 11 which is encased in the lancet body to prevent the needle from moving forwardly when the cap is removed. As shown in Figure 5 it is an abrupt step, but it could be a tapered transition.

In one example of the manufacture of the lancet, a supply of needle blanks of the appropriate length but of constant diameter is fed to an automated centreless cylindrical grinding machine which is set up to grind the needle blanks to provide the reduced diameter tip portion 10. These rotationally symmetric intermediate blanks are then supplied to a grinding machine to form the needle point or tip 4 (typically by grinding three flats). The formed needles are then delivered to a moulding machine where they are held within the mould cavity by mould pins (not shown). The body, and cap if provided, is then moulded around the needle in plastics material. Finally, the completed lancet is ejected from the mould.

## Claims

1. A lancet for a skin pricker, the lancet having a needle (3) encased, apart from its tip, in a body (1) of plastics material, **characterised in that** the needle (3) has a larger diameter portion (9) of generally circular cross-section within the lancet body (1) but with a coaxial reduction in diameter before emerging at the tip (4), to provide a smaller diameter portion (10) of generally circular cross-section, and **in that** the larger diameter portion of the needle has a diameter in the range 0.6 to 1.0 mm.

2. A lancet as claimed in Claim 1, wherein the reduction in diameter is by an abrupt step (11).

3. A lancet as claimed in Claim 1, wherein the reduction in diameter is by a tapered shoulder.

4. A lancet as claimed in Claim 1, 2 or 3, wherein a breakaway cap (5) is integrally moulded with the body (1) to encase the needle tip.

5. A lancet as claimed in any of the preceding Claims, wherein the larger diameter portion of the needle has a diameter of 0.8 mm.

6. A lancet as claimed in any preceding claim, wherein the reduced diameter portion (10) of the needle has a diameter in the range 0.2 to 0.5 mm.

7. A lancet as claimed in Claim 7, wherein the reduced diameter portion of the needle has a diameter in the range 0.3 to 0.4 mm.

8. A method of producing a lancet for a skin pricker, the method comprising:
providing a generally cylindrical needle blank,
reducing the diameter of one end portion (10) of the blank,
forming a point on the free end or tip (4) of said one end portion (10), thus creating a needle,
introducing the needle into a mould, and
introducing plastics material into said mould to form a lancet body around the needle apart from its tip, **characterised in that** the reduced diameter portion (10) is of generally circular cross-section and coaxial with the larger diameter portion, and **in that** the reduced diameter portion has a diameter in the range from 0.6 mm to 1.0 mm.

9. A method as claimed in Claim 8, wherein the reduction of said one end portion of the blank is by cylindrical grinding.

10. A method as claimed in Claim 8, wherein the reduction of said one end portion of the blank is by rolling.

11. A method as claimed in Claim 8, wherein the reduction of said one end portion of the blank is by turning.

## Patentansprüche

1. Lanzette für einen Hautstecher, mit einer von einem Körper (1) aus Kunststoffmaterial mit Ausnahme ihrer Spitze umgebenen Nadel (3), **dadurch gekennzeichnet, daß** die Nadel (3) einen größeren Durchmesserteil (9) von im allgemeinen kreisrunden Querschnitt in dem Lanzettenkörper (1) aufweist, jedoch mit einer koaxialen Verkleinerung des Durchmessers vor dem Austritt an der Spitze (4) versehen ist, der einen kleineren Durchmesserteil (10) von im allgemeinen kreisrunden Querschnitt bildet, und daß der größere Querschnittsteil der Nadel einen Durchmesser im Bereich von 0,6 bis 1,0 mm hat.

2. Lanzette nach Anspruch 1, **dadurch gekennzeichnet, daß** die Verringerung des Querschnitts durch eine abrupte Stufe (11) geschieht.

3. Lanzette nach Anspruch 1, **dadurch gekennzeichnet, daß** die Verringerung des Querschnitts durch eine abgeschrägte Schulter erfolgt.

4. Lanzette nach Anspruch 1, 2 und 3, **dadurch gekennzeichnet, daß** eine Wegbrechkappe (5) mit dem Körper (1) zu einem Stück spritzgegossen ist, um die Nadelspitze zu umhüllen.

5. Lanzette nach einem der vorhergehenden Ansprüche, **dadurch gekennzeichnet, daß** der größere Durchmesserteil der Nadel einen Durchmesser von 0,8 mm hat.

6. Lanzette nach einem der vorhergehenden Ansprüche, **dadurch gekennzeichnet, daß** der reduzierte Durchmesserteil (10) der Nadel einen Durchmesser im Bereich von 0,2 bis 0,5 mm hat.

7. Lanzette nach Anspruch 7, **dadurch gekennzeichnet, daß** der reduzierte Durchmesserteil der Nadel einen Durchmesser im Bereich von 0,3 bis 0,4 mm hat.

8. Verfahren zur Herstellung einer Lanzette für einen Hautstecher, umfassend die Schritte: Schaffung eines im allgemeinen zylindrischen Nadelrohlings, Verringerung des Durchmessers des einen Endteils (10) des Rohlings, Formung eines Punktes auf dem freien Ende oder der Spitze des einen Endteils (10), um **dadurch** eine Nadel zu schaffen, Einführen der Nadel in eine Gießform und Einführen von Kunststoffmaterial in diese Gießform, um rund um die Nadel neben ihrer Spitze einen Lanzettenkörper zu bilden, **dadurch gekennzeichnet, daß** der verringerte Durchmesserteil (10) einen im allgemeinen kreisrunden Querschnitt hat und koaxial zu dem größeren Durchmesserteil liegt, und daß der verringerte Durchmesserteil einen Durchmesser im Bereich von 0,6 mm bis 1,0 mm aufweist.

9. Verfahren nach Anspruch 8, **dadurch gekennzeichnet, daß** die Verringerung des einen Endteils des Rohlings durch Schleifen eines Zylinders erfolgt.

10. Verfahren nach Anspruch 8, **dadurch gekennzeichnet, daß** die Verringerung des einen Endteils des Rohlings durch Walzen erfolgt.

11. Verfahren nach Anspruch 8, **dadurch gekennzeichnet, daß** die Verringerung des einen Endteils des Rohlings durch Drehen erfolgt.

## Revendications

1. - Lancette pour un dispositif à piquer la peau, la lancette ayant une aiguille (3) encastrée, à l'exception de sa pointe, dans un corps (1) en matière plastique, **caractérisée par le fait que** l'aiguille (3) a une partie (9) de plus grand diamètre, de section transversale généralement circulaire à l'intérieur du corps de lancette (1) mais avec une réduction coaxiale de diamètre avant d'émerger à la pointe (4), pour fournir une partie (10) de plus petit diamètre, de section transversale généralement circulaire, et **par le fait que** la partie de plus grand diamètre de l'aiguille a un diamètre dans la plage de 0,6 à 1,0 mm.

2. - Lancette selon la revendication 1, dans laquelle la réduction de diamètre s'effectue par un gradin abrupt (11).

3. - Lancette selon la revendication 1, dans laquelle la réduction en diamètre s'effectue par un épaulement conique.

4. - Lancette selon l'une des revendications 1, 2 ou 3, dans laquelle un capuchon détachable (5) est moulé d'un seul tenant avec le corps (1) pour encastrer la pointe d'aiguille.

5. - Lancette selon l'une quelconque des revendications précédentes, dans laquelle la partie de plus grand diamètre de l'aiguille a un diamètre de 0,8 mm.

6. - Lancette selon l'une quelconque des revendications précédentes, dans laquelle la partie (10) de diamètre réduit de l'aiguille a un diamètre dans la plage de 0,2 à 0,5 mm.

7. - Lancette selon la revendication 7, dans laquelle la partie de diamètre réduit de l'aiguille a un diamètre dans la plage de 0,3 à 0,4 mm.

8. - Procédé de fabrication d'une lancette pour un dispositif à piquer la peau, le procédé comprenant les étapes consistant à :
- se procurer une ébauche d'aiguille généralement cylindrique ;
- réduire le diamètre d'une partie d'extrémité (10) de l'ébauche ;
- former une pointe sur l'extrémité libre ou pointe (4) de ladite partie d'extrémité précitée (10), créant ainsi une aiguille ;
- introduire l'aiguille dans un moule ; et
- introduire de la matière plastique dans ledit moule pour former un corps de lancette autour de l'aiguille à l'exception de sa pointe,
**caractérisé par le fait que** la partie de diamètre réduit (10) est de section transversale généralement circulaire et coaxiale avec la partie de plus grand diamètre, et **par le fait que** la partie de diamètre réduit a un diamètre dans la plage de 0,6 mm à 1,0 mm.

9. - Procédé selon la revendication 8, dans lequel la réduction de ladite partie d'extrémité précitée de l'ébauche s'effectue par meulage cylindrique.

10. - Procédé selon la revendication 8, dans lequel la réduction de ladite partie d'extrémité précitée de l'ébauche s'effectue par laminage.

11. - Procédé selon la revendication 8, dans lequel la réduction de ladite partie d'extrémité précitée de l'ébauche s'effectue par tournage.
